# EUROPEAN PATENT SPECIFICATION

(11) **EP 1 184 041 B1**
(45) Date of publication and mention of the grant of the patent: **03.03.2010**
(21) Application number: 00925691.8
(22) Date of filing: 16.05.2000
(51) Int. Cl.: A61L 29/06, A61M 25/10, A61M 25/00

(54) **METHOD FOR FIXEDLY MOUNTING A BALLOON ON A CATHETER TUBE**
VERFAHREN ZUR BEFESTIGUNG EINES BALLONS AN EINEM KATHETERSCHLAUCH
PROCEDE DE FIXATION D'UN BALLONNET A UNE TUBULURE DE CATHETER

(30) Priority: 16.05.1999 JP 17147399; 15.05.2000 JP 2000180528
(43) Date of publication of application: 06.03.2002
(73) Proprietor: YS, Medical Co., Ltd., Kawageo-city, Saitama 350-1141 (JP)
(72) Inventor: YOSHINO, Yosuke, Saitama 350-1141 (JP); KIMURA, Masanori, Hirakawa Fewteck K.K., Sashima-gun, Ibaraki 306-0232 (JP); TAKEZAWA, Takashi, Hirakawa Fewteck K.K., Sashima-gun, Ibaraki 306-0232 (JP)
(74) Representative: Beetz & Partner
(86) International application number: PCT/JP2000/003141
(87) International publication number: WO 2000/069487

(56) References cited:
- WO-A-95/25560
- WO-A-98/39044
- JP-A- 5 168 694
- JP-A- 5 184 664
- JP-A- 53 069 488
- US-A- 4 154 244
- US-A- 5 562 720
- US-A- 5 718 861

## Description

The present invention relates to a method of fixedly mounting a ballon on a catheter tube. The balloon catheter is such a medical instrument that is inserted into the blood vessel and made stay there, and blocks the vascular stream by expanding the balloon.

Recently, a medical treatment technique for keeping a long-term cyclic operation for injecting, aspirating and recovering intense anti-cancer drugs is put into practice and innovative results are achieved in the actual medical field, in which the arterial flow to the deseased parts of the internal organs and the venous flow from those parts and organs are blocked by using the balloon catheter, the arterial flow and the venous flow for the predetermined organ is blocked out from the whole-body cardiovascular system, and the anti-cancer drugs in high concentration are injected at the arterial blood vessel into the predetermined organ with its diseased parts being isolated as well as the anti-cancer drugs flowing out from the venous blood vessel (or the portal vein) are exteriorized. In general, this is called an anti-cancer drug perfusion system, which allows the highly concentrated anti-cancer drugs to be exposed to the cancer part and enables a systematic operation for injecting and aspirating the anti-cancer drugs because the anti-cancer drugs may not circulate in the whole body through the heart.

Because it is, however, required to insert and stay the catheter, for example, directly from the femoral artery to the arterial part of the diseased part of the internal organ, there may be the possibility where the balloon part of the catheter may make damage onto the arterial vascular wall in the system during the inserting operation in case that the balloon part itself is hard when non-expanded. In general, it is well known that the internal arterial vascular wall is more vulnerable than the internal venous vascular wall is. The larger the diameter of the catheter tube is, the more the risk of giving a damage to the inner vascular wall is. In the conventional balloon catheter, the balloon in its non-expanded state is made very "solid" in order to establish mainly an accurate sealing ability when the balloon expands, which may not infrequently result in damages in the inner vascular wall by the balloon part during the process for inserting the catheter to the arterial blood vessel at the diseased part. In addition, as for silicon and polyurethane synthetic rubbers used conventionally, their restoring force is so week after expansion work that any damage occurs in the blood vessel when removing the catheter. Latex materials cannot provide larger expansion coefficient as well as those materials may arise thrombus due to their direct contact to the blood itself. This thrombus may arise when the inner vascular wall is damaged.

The international patent application WO 98/39044 discloses a catheter balloon with an improved elastic response and a reduced longitudinal expansion, comprising a SEBS block copolymer, wherein the balloon is made by extrusion and then stretched in a longitudinal direction.

According to the present invention a method of fixedly mounting a balloon on a catheter tube, one or more balloons being arranged close to an insertion top edge of the catheter tube and arranged separately in an axial direction, is provided. The method comprises the steps of providing a catheter tube on which said balloon can be located and fixedly mounted at a designate position and placing the balloon approximately shaped in a cylinder at a non-expanded state on the periphery of said catheter tube adjacent to the predetermined position of said balloon ; The method of the present invention is characterized in it comprises the further step of fixedly mounting both ends of said balloon in the axial direction at an elongated state in the axial direction so as to cover said predetermined position of said catheter tube.

With the method of the present invention a balloon catheter is provided wich is preferably used for the anticancer drug perfusion systems, that is, composed of materials which could not give a damage to the inner vascular wall with its balloon part being in the occlusion state when the catheter is inserted and fixed at a predetermined position in the blood vessel, and that provide a reliable occlusion ability when expanded.

With the method of the present invention a larger occlusion volume is provided even when a balloon is mounted on a catheter tube with smaller diameter.

The balloon catheter is inserted in the blood vessel. Furthermore, a balloon can be provided for blocking the vascular stream in the blood vessel when expanded, in which the material used for the balloon is flexible especially when occluding the blood vessel and being extracted for itself. The material can be selected from styrene group SBS, SIS, SEBS and/or SEPS thermoplastic elastomer materials. The tensile rupture ductility is between 800% and 2000%, and the used material is selected from materials which do not make thrombus when contacting to the blood. Those values for the tensile rupture ductility exceed the maximum generic ductility of the balloon materials themselves.

As for the elastomer materials applied to the balloon materials used for the balloon catheter, latex or silicon resin in rubber materials, or polyolefin, polyester, polyurethane, polyamide, or styrene compounds etc. in plastic materials are typically used. As for the materials used for flexible balloons for occluding a blood vessel, elastic latex, silicon resin or polyurethane resin is generally used.

Those balloon materials have their own mechanical characteristics with respect to the maximum elongation percentage (tensile rupture ductility), which include the following typical balloon materials; the range of ductility of SB group is approximately from 300% to 1000%, the range of ductility of SEBS group is approximately from 500% to 1000%, the range of ductility of natural rubber is approximetaly from 300% to 900%, the range of ductility of silicon rubber is approximately from 230% to 900%, the range of ductility of olefin group is approximately from 300% to 600%, the range of ductility of vinyl chloride group is approximately from 400% to 500%, the range of ductility of polyurethane group is approximately from 300% to 800%, the range of ductility of polyester group is approximately from 380% to 420%, and the range of ductility of polyamide group is approximately from 200% to 400%. The upperbound for the ductility of general flexible materials is approximately 100%

The expansion characteristics of typical elastic balloon materials such as latex, SEBS, silicon rubber, etc. are described as examples below, in which the reference balloon is formed with its inner diameter of the balloon being 6 mm and its wall thickness being 0.5 mm.

Water was filled into the balloon to expand it. The balloon made of latex was broken at the full limits of 700% elongation and 75 ml injection water volume, the balloon made of SEBS was broken at the full limits of approximately 830% elongation and approximately 130 ml injection water volume, and the balloon made of silicon was broken at the full limits of 700% elongation and approximately 120 ml injection water volume. As apparent from those experimental evidences, the full limits of the elongation of the balloon are almost identical to those for the individual raw materials.

The above fact explains that, if applying the balloon made of the existing balloon materials simply to the catheter, the maximum elongation characteristics is bound by the intrinsic elongation characteristics of the existing materials.

A highly flexible, highly elastic and highly extendable diameter balloon catheter is provided. The balloon is expected to have higher flexibility in order to prevent the non-expanded balloon from damaging the blood vessel during the balloon moving operation when positioning and fixing the balloon mounted on the catheter tube at the predetermined position in the blood vessel. After completing the medical treatment, it is required to release the expansion operation for the ballon and draw out the catheter from the blood vessel in the human body. At this time, the balloon is expected to be returned to the non-expanded state or substantially original state. Therefore, it is desirable that the balloon itself is highly elastic. In addition, the diamter of the catheter is required to be smaller enough compared with the inner diameter of the inner wall of the blood vessel so that the catheter may move easily in the blood vessel. Therefore, the balloon mounted on such a catheter with small diameter must have higher expandability and it must block the vascular stream absolutely when expanded. For the additional condition, the materials used for the balloon should not be those causing the blood contacting to them to arise any thrombi. The thrombus may occur due to the damage of the inner vascular wall by the balloon.

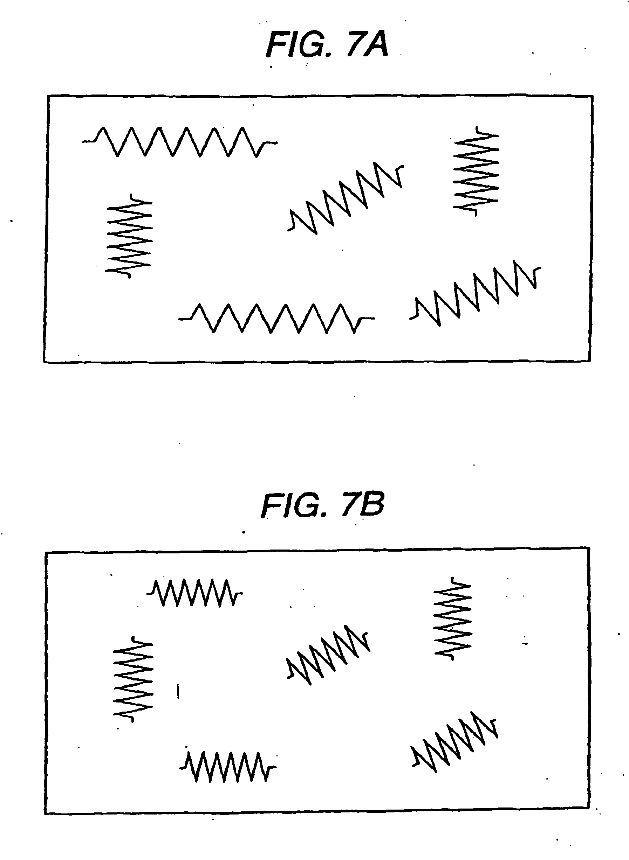

A balloon is formed generally by using a physical process including melting, molding and cooling for thermoplastic materials. Figs. 7A to D illustrate models of macro-molecular elongation for the extrusion molding, the melting molding with a mold the injection molding, and the dip molding by the liquid solution, respectively. The graphical symbols in the figure schematically represent the elongation percentage of the macro-molecular chain as a visual image of the cross section of the coil.

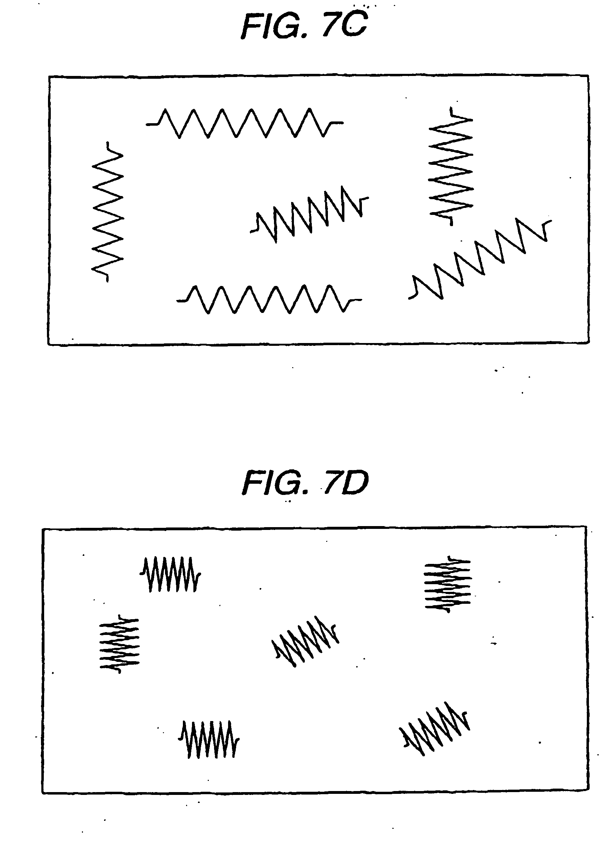

As the molten material flows through the cavity into the mold in the balloon fabricated by the injection molding shown in Fig. 7C, a deviation in the macro-molecular chain arises along the injection flow, and the elongation characteristic greatly depends upon the direction. As shown in this figure, the coils arranged in the individual directions show their own elongation states, and the degree of elongation in those directions is restrained.

Fig. 7A shows a case of the extrusion molding. As the molding material flows with its orientation in the direction of the extrusion work and is solidified, the molecular chain extends in the longitudinal direction. Owing to this characteristic in the molding work, the elongation percentage of the coil in the longitudinal direction is restrained.

In the case shown in Fig. 7B, as the molten material is shaped in the mold, its molecular structure is wholly uniform, and directional dependency does not occur in general, and thus, uniform elongation characteristics can be obtained.

In the dip forming using the liquid solution shown in Fig. 7D, as the polymer is solved in the solvent, and the polymer is coated on the surface of the mold, and then, the solvent is removed, more uniform elongation characteristics can be obtained.

### Brief Description of the Drawings

- Fig. 1: is a schematic diagram of a first example of the fabrication apparatus for the balloon of the balloon catheter.
- Fig. 2: is a partial side view of the balloon formation part of the mold used in the apparatus shown in Fig. 1.
- Fig. 3: is a schematic diagram of a second example of the fabrication apparatus for the balloon of the balloon catheter.
- Fig. 4: illustrates that the balloon catheter is inserted into the hepatic artery, and that the balloon is expanded for blocking the vascular stream.
- Fig. 5: is a schematic diagram of the balloon protection tube used with the balloon catheter in association with the sheath and the balloon catheter.
- Fig. 6: is a front view of the insertion hole of the sheath in which the balloon catheter is inserted.
- Figs. 7A: to 7D show the illustrative models of macro-molecular structure expanded by the injection molding process, the extrusion molding process, the melting molding process with mold tools and the dip forming process with liquid solutions, respectively, used for the better understanding of the present invention.
- Fig. 8: shows an example for obtaining the balloon with highly expandable diameter. .
- Figs. 9A: and 9B show the conventional mount method of the balloon and the mount method applying the principles of the present invention, respectively.
- Fig. 10: shows a table of elongation percentage of SEBS bulk balloon according to the principles of the present invention.
- Fig. 11: shows an illustrative model for providing a theoretical understanding of the principles of the present invention.
- Fig. 12: shows a table of measured values for the principles of the present invention to be applicable to the materials other than SEBS materials.
- Fig. 13: shows an embodiment facilitating the mounting of the bulk balloon at the catheter.

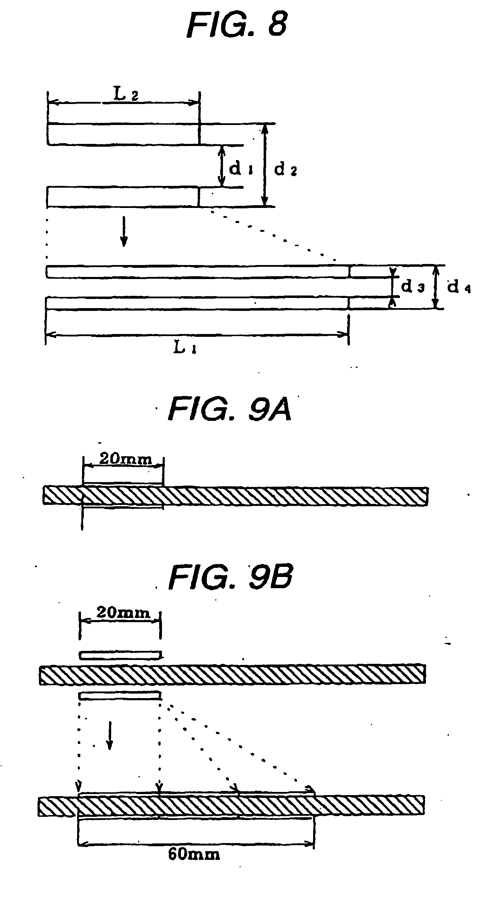

Fig. 8 shows an example for obtaining a balloon having large expandable diameter. In this embodiment, as described in detail below, the balloon is mounted on the catheter designed to have a size equal to the desired tube outer diameter, with the balloon being elongated in the longitudinal direction. In this mounting operation, the structure of the catheter balloon in the product is determined at first, and then, the stretch ratio at the fixing of the balloon is determined. Suppose that L2 is the length of the balloon before stretching, L1 is the length of the balloon after stretching, d1 is the inner diameter of the balloon before stretching, d2 is the outer diameter of the balloon after stretching, d3 is the inner diameter of the balloon after stretching and that d4 is the outer diameter of the balloon after stretching, the elongation ratio ε is defined by L2/L1, the inner diameter d 1 of the balloon before stretching can be represented by the product of the square root of the reciprocal of elongation ratio ε and the inner diameter d3 of the balloon after stretching, and the outer diameter d2 of the balloon before stretching can be represented by the product of the square root of the reciprocal of elongation ratio ε and the outer diameter 94 of the balloon after stretching. Those relations provide design formulae for the bulk balloon (balloon formed after stretching). This is effective for the design of balloons because it is necessary to consider the size of the catheter the outer diameter of the catheter tube at first.

Fig. 9 A illustrates an ordinary mounting method in which the balloon is mounted onto the catheter tube without stretching. This is an embodiment in which SEBS balloon with 2.8 mm balloon outer diameter, 2 mm balloon inner diameter, and 0.4 mm thickness and 20 mm length is mounted on 6 French catheter tube (2 mm outer diameter). Fig. 9 B is a mounting example of the balloon in which the elongation multiplying factor is 200%, that is, the balloon composed of the same material and structure as in Fig. 9 A is stretched to the length L1 = 60 mm, which is equeal to twice as long as the original length of the balloon, excluding the original length of the balloon itself. In the design of those bulk balloons, it is necessary to determine the outer diameter of the catheter tube to be mounted with the balloon at first. This value for the outer diameter can be substantially equal to the internal diameter d3 after balloon stretching. Next, the balloon fixing length is determined. In this example, the fixing length L2 is 20 mm. Thus, the elongation ratio ε = L2/L1 is 1/3. In this example, the calculation value of the inner diameter d 1 of the balloon before stretching is estimated to be 3.46 mm by applying the above design formula. The outer diameter of the balloon before stretching is estimated to be 4.85 mm by assuming that the thickness of the balloon after stretching is 0.4 mm which is equal to the thickness before stretching, and its outer diameter is 2.8 mm.

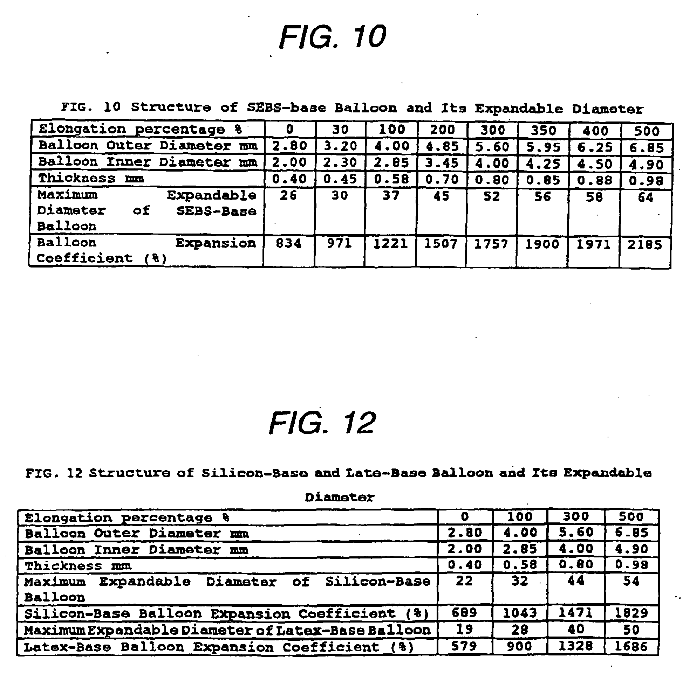

Fig. 10 shows a table including the measured values for the maximum diameter (mm) and the stretching degree (%) of the balloon mounted on the catheter tube (6 French) shown in the above example, with the SEBS bulk balloon being fixed at the elongation percentages, 0 % (for the non-stretched length, 20 mm), 30 % (stretched to 26 mm), 100 % (stretched to 40 mm), 200 % (stretched to 60 mm), 300 % (stretched to 80 mm), 350 % (stretched to 90 mm), 400 % (stretched to 100 mm), and 500 % (stretched to 120 mm). As apparent from this measurement results, in case that the bulk balloon is mounted in its elongated state according to the present invention, its elongation percentage highly exceeds the maximum elongation percentage (at most 1000%) specific to the materials without stretching. Thus, the highly expandable diameter characteristic of the balloon which cannot be provided by the conventional balloon can be obtained.

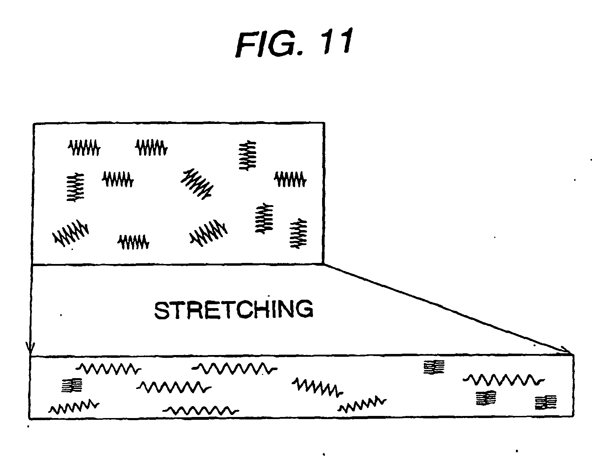

Fig. 11 illustrates one of the theoretical understanding of the highly expandable diameter characteristics in the balloon. The upper part of Fig. 11 is a schematic diagram of the macro-molecular chain structure in the bulk balloon before stretching, in which the graphical chain symbol represents a tension spring as a model of the macro-molecular cross-section. Though the macro-molecular chain in the longitudinal direction is stretched as shown in the lower part in Fig. 11, the macro-molecular chain in the radial direction is not stretched, and thus, it is supposed that the expandability characteristic in the radial direction of the bulk balloon may be maintained after stretching. Therefore, by making the diameter of the bulk balloon larger than the diameter of the catheter tube on which the bulk balloon is mounted, and fixing the bulk balloon with its longitudinal length stretched, the diameter of the bulk balloon can be expanded up to its original expandable diameter.

Though the above example refers to SEBS balloon material, the present invention can be applied to other materials. Fig. 12 shows the measured values for the expandability characteristic for the elongation percentages, 0 %, 100 %, 300 % and 500 % in the balloons made of silicon resin and latex. As apparent from those measured values, by applying the stretching processing according to the present invention to the balloon materials used conventionally, the expandability far higher than the maximum expandability originally specific to the used materials themselves can be provided.

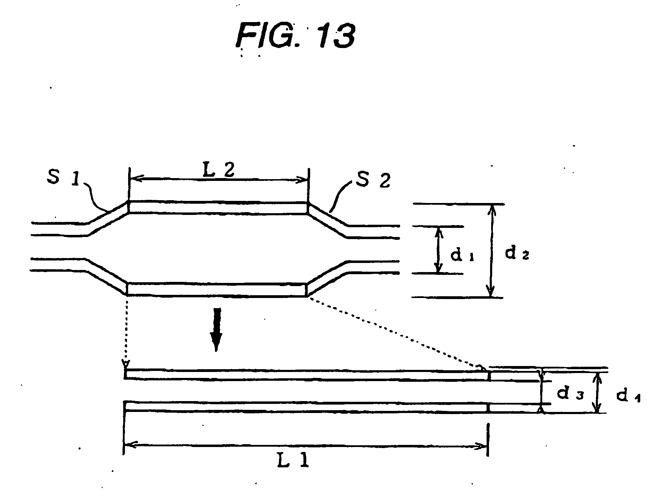

Fig. 13 illustrates an example of forming a bulk balloon which enables the above mentioned bulk balloon easily mounted with the catheter tube. The bulk balloon before stretching has sloped parts S1 and S2, and those sloped parts substantially contact firmly to the catheter tube after stretching the balloon and mounting it on the catheter tube (not shown), and form a bonded part of the balloon for the catheter.

According to the present invention, a manufacturing method for the balloon catheter can be provided, in which the above described balloon can be formed by dipping a mold having a predetermined diameter plural times into the fused solution or liquid solution containing the material selected from styrene group SBS, SIS, SEBS, SEPS, thermoplastic elastomer materials from the view point of flexibility and thrombus.

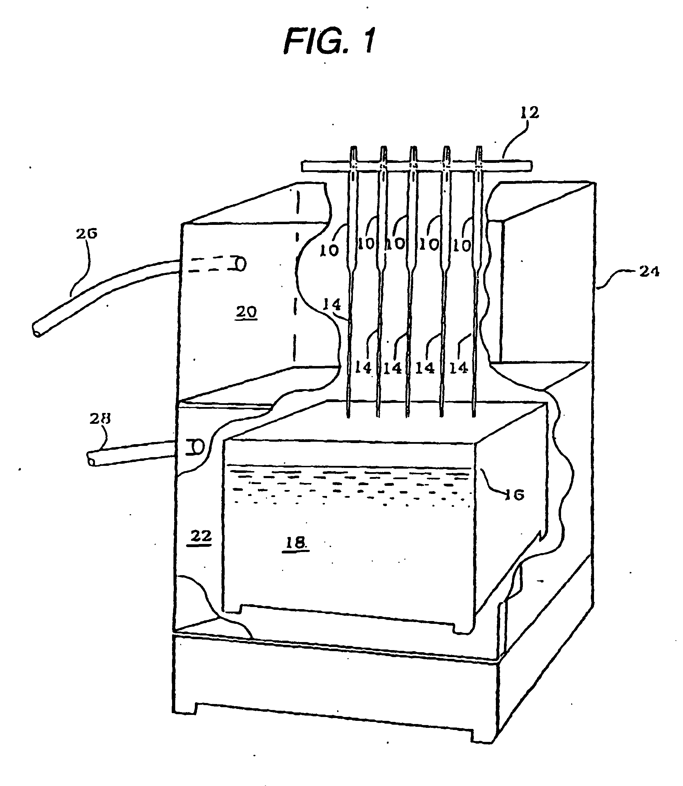

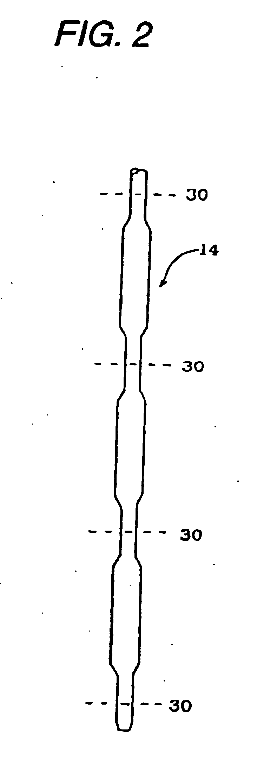

Fig. 1 shows a first example for fabricating the balloon for the balloon catheter. In Fig. 1, the components 10 are plural molds, for example, made of stainless steel, supported by the jig 12. As shown also in Fig. 2, the mold 10 has a balloon formation part 14. The jig 12 is linked to an elevating mechanism not shown which moves upward or downward plural molds 10 simultaneously. The balloon formation part 14 is dipped into the molten solution 18 of the balloon materials contained in the molten solution bath 16 at the lowest position of the mold. The molten solution bath 16 is contained in the lower compartment 22 of the housing 24 composed of the upper compartment 20 and the lower compartment 22. The components 26 and 28 are tubes or pipes for supplying, for example, nitrogen gas, to the upper and lower compartments, respectively. This gas cools down the balloon materials in the balloon formation part of the elevated mold at the upper compartment 20, and it operates as oxidation inhibitor for the molten solution 18 of the balloon materials contained in the molten solution bath 16 at the lower compartment 22.

The molten solution of the balloon materials contains the following balloon materials melted at the selected temperature between 150°C and 250 °C. The balloon material is selected from the thermoplastic elastomer materials, especially the styrene-group thermoplastic elastomer materials, including styrene-butadiene-styrene (SBS), styrene-polyisoprene-styrene (SPS), styrene-polyethylene/polybutylene-styrene (SEBS), and styrenepolyethylene/ propylene-styrene (SEPS) structures. And furthermore, the material having 800 % to 2000 % tensile rupture ductility is selected from those materials.

Most materials are put on the market. Especially, SBS-group and SEBS-group "elastomer AR" available from Aron Chemical Ltd., SBS-group "JSRTR" available from Japan Synthetic Rubber Co., Ltd., SIS-group "JSRSIS" available from Japan Synthetic Rubber Co., Ltd., SIS-group "Hypler" available from Kuraray Co., Ltd., SEBS-group "Kraton G" available from Shell Oil Co., SEBS-group "Ruberon" available from Mitsubishi Petrochemical Co., Ltd., and SEPS-group "Scepton" available from Kuraray Co., Ltd., satisfy the above requirements for the balloon of the catheter as well as the balloon made from those materials does not cause the thrombus due to its direct contact to the blood in the blood vessel.

The balloon made from the the above-described materials is extremely soft itself, and could never damage the inner vascular wall during the movement of the catheter especially in the narrow blood vessel.

In Fig. 1, the balloon formation part 14 of the mold 10 is dipped into the molten material solution 18 for a predetermined period of time, and drawn out and its liquid component is evaporated, which is repeated in a predetermined number of times. For example, in case of the outer diameter of the catheter being 5 and 8 French (FC) (equal to 1.6 mm and 2.7 mm, respectively), the balloons with their thickness being approximately 0.1mm and 0.14 mm, respectively, are formed, and the balloon with its thickness being 0.31 mm is formed for the outer diameter of the catheter being 12 French (equal to 3.9 mm), which are proved to be acceptable as the balloon for the catheter used for blocking the vascular stream in the anti-cancer drug perfusion system.

Fig. 2 is a magnified view of the balloon formation part of the mold 10. In completing plural repetitive cycles of dipping and evaporation operation, the long balloon material formed on the surface of the balloon forming part is cut off at the position 30 and the individual pieces are extracted separately, and thus plural balloon parts are obtained from a single mold 10. In Fig. 2, the balloon part formed has a shape in which the diameter of its center part is slightly larger than the diameter of its both ends to which the catheter is coupled. This shape is aimed to cope with the diameter expansion, and the shape of the balloon formation part 14 may be a straight cylinder if there is no need for considering the diameter expansion.

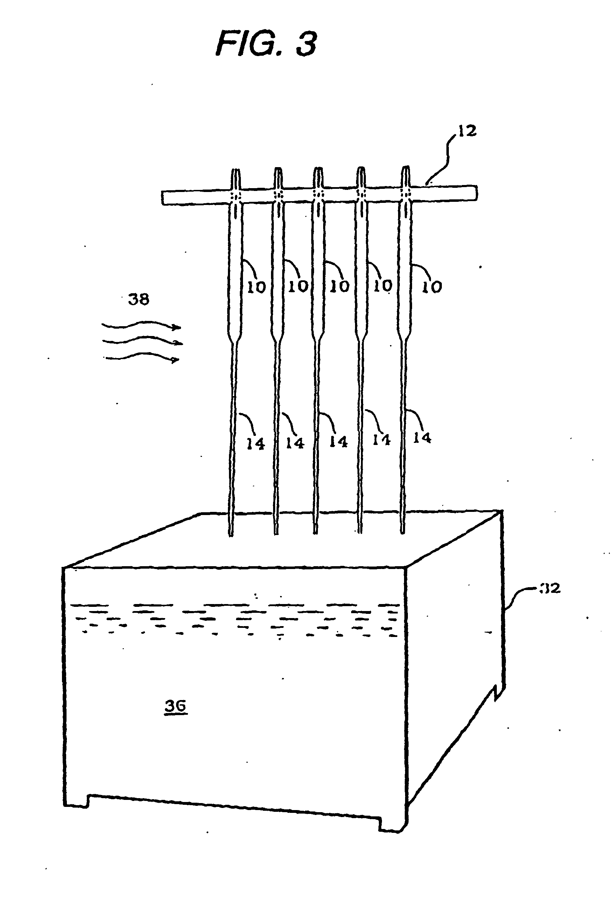

Fig. 3 shows a second example of the balloon fabrication apparatus. As in the embodiment shown in Fig. 1, plural molds 10 having the balloon formation parts 14 are supported by the jig 12 used as a support device so as to enable to move upward and downward. The balloon formation part 14 is dipped into the liquid bath 36 containing the styrene-group thermoplastic elastomer materials dissolved with organic solvent such as thinner, triol-group, toluene-group and benzene-group, and drawn up to the predetermined uprising position after a predetermined period of time where the balloon formation part is made dried, for example, by hot air 38, and this dipping and evaporation cycle is repeated in a predetermined number of times. So far, it is aimed that the balloon part having the above-described thickness can be obtained in completing these repetitive cycles.

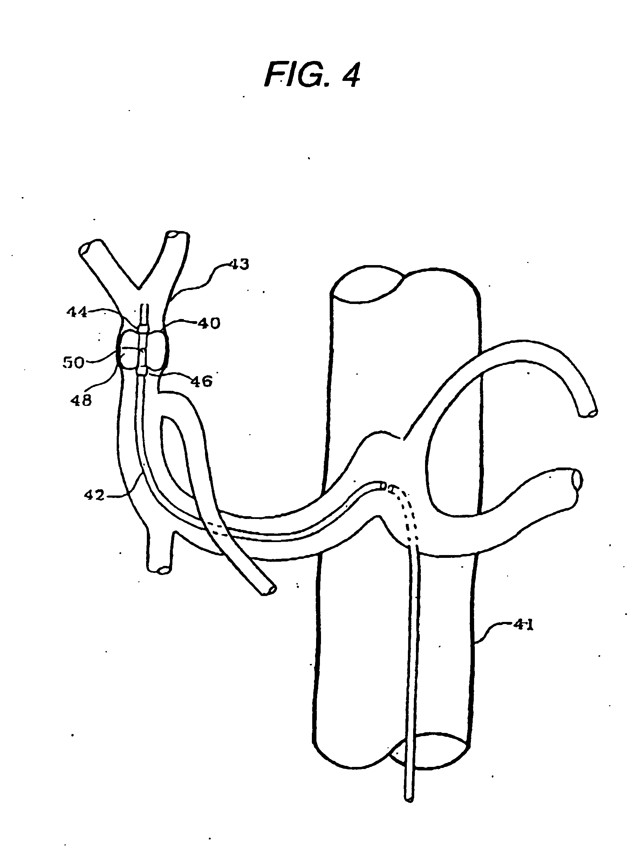

Fig. 4 illustrates that the catheter 42 with a balloon 40 at its top end is inserted, for example, from the femoral artery through the aorta 41 to the predetermined position in the hepatic artery 43, where the balloon is expanded for blocking the vascular stream. The balloon 40 is fixed at the periphery of the catheter (catheter tube) 42 with its both ends 44 and 46, and an expansion cavity 48 is formed when expanded. For this purpose, an expansion fluid aperture 50 is formed for the expansion fluid passage way (not shown) inside and along the catheter. The balloon formed by the above mentioned material with the above mentioned thickness and fixed at the catheter having the above mentioned French index can be expanded up to the diameter approximately 15 times as large as the outer diameter of the catheter. Thus, as far as the balloon is used in such a practical use that its outer diameter expands up to approximately several times as large as the outer diameter of the catheter, its expanded outer diameter can come back to its initial size.

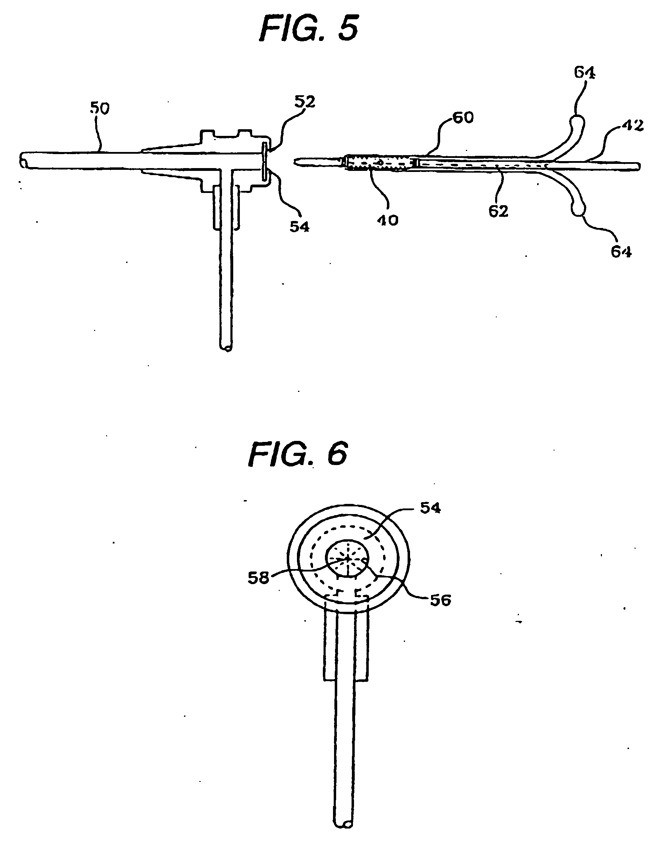

When the catheter is inserted into the blood vessel, in general, a catheter introducer comprising a sheath (external cylinder), an internal cylinder and a guide wire, if necessary, is at the start inserted into the blood vessel, and then, the internal cylinder is extracted when the catheter introducer reaches a predetermined position in the blood vessel, and the catheter is inserted into the sheath, and finally, the catheter is guided by the guide wire and inserted to the specified position in the blood vessel. Fig. 5 illustrates that the catheter 42 is inserted from the insertion part 52 of the sheath 50. As shown also in Fig. 6, the insertion part 52 has a seal film member 54 typically made of rubber materials for preventing the air from penetrating into the sheath. Cut lines 56 are originally formed at the seal film member 54, and by inserting the top edge of the catheter through its center 58, the catheter can be inserted inside the sheath while the sealed condition is maintained. With this configuration, the conventional balloon catheter can be used without any trouble. However, as a soft and thin material is used in the catheter of the present invention that the balloon 40 may not damage the inner vascular wall, the balloon 40 itself may be damaged when the balloon part of the catheter passes through the seal film member. In order to solve this problem, the balloon part of the catheter is covered by the protection tube 60 as shown in Fig. 5 in the present invention. Starting from the state shown in the figure, when the catheter is inserted into the sheath and after the balloon part passes through the seal film member, the protection tube 60 is made move in the axial direction backward to the position where it may not interfere with the catheter, or the protection tube 60 is removed at any position on the catheter (tube) part behind the balloon to which the protection tube 60 is made move while the sealed condition by the film member 54 can be maintained. For the purpose of the latter case mentioned above, a couple of members 64 for separating the protection tube 60 into two pieces between which formed is the cut line 62 for facilitating the separation work. The requirements for the protection tube includes that the air tightness inside the sheath can be guaranteed, and that the tube should not damage the balloon 40 by itself when moving backward in the axial direction in relative to the catheter.

In order to establish the hermetic seal, the head end of the tube 60 is hermetically contacted to the catheter coupling part of the balloon as shown in the figure. It is required that the hermetic seal condition inside the tube 60 is established by the time when the protection tube is moved backward toward the back end of the catheter in its axial direction and the seal coupling between the catheter and the film member 54 is obtained at the position behind the balloon part of the catheter. In order to meet this requirement, it is allowed that the similar member (not shown) to the seal film member 54 is placed at the back end of the tube.

The problem regarding to the damage to the balloon by the tube when the tube is moved backward toward the back end of the catheter can be solved by shaping the tope edge of the tube 60 so as to be slightly tapered as shown in the figure, optimizing the size of the aperture at its top edge, and making the inner surface of the aperture smooth. This structure makes it easier for the balloon part of the catheter to insert hermetically into the seal film member.

## Claims

1. A method of fixedly mounting a balloon (40) on a catheter tube (42), one or more balloons (40) being arranged close to an insertion top edge of the catheter tube (42) and arranged separately in an axial direction, comprising the steps of:
- providing a catheter tube (42) on which said balloon (40) can be located and fixedly mounted at a designate position;
- placing the balloon (40) approximately shaped in a cylinder at a non-expanded state on the periphery of said catheter tube (42) adjacent to the predetermined position of said balloon (40);
**characterized in that** the method comprises the further step of
- fixedly mounting both ends of said balloon (40) in the axial direction at an elongated state in the axial direction so as to cover said predetermined position of said catheter tube (42).

2. Method according to claim 1, wherein
- said balloon (40) has an axial-direction both ends part having an inner diameter substantially equal to an outer diameter of a mounted catheter before stretching and an intermediate part having an outer diameter substantially larger than the outer diameter of said axial direction both ends part before stretching it; and
- said axial direction both ends part is fixedly mounted on said catheter tube (42) so that an approximately uniform cylinder-shaped balloon (40) can be formed by stretching said intermediate part when stretching in the axial direction.

3. Method according to claim 1, wherein
- said balloon (40) has an axial-direction both ends part having an inner diameter substantially equal to the outer diameter of a mounted catheter before axial-direction stretching and an intermediate part having an inner diameter substantially larger than the outer diameter of said axial direction both ends part before stretching ; and wherein
- said axial direction both ends part is fixedly mounted on said catheter tube (42) so that an approximately uniform cylinder-shaped balloon (40) can be formed by stretching said intermediate part when stretching it.

## Patentansprüche

1. Verfahren für das feste Montieren eines Ballons (40) auf einem Katheterschlauch (42), bei dem ein oder mehrere Ballons (40) in der Nähe der oberen Insertionsspitze des Katheterschlauches (42) angeordnet werden und getrennt voneinander in axialer Richtung angeordnet werden, das folgende Schritte umfasst:
- Bereitstellen eines Katheterschlauches (42), auf dem der Ballon (40) angeordnet und in einer gewünschten Position fest montiert werden kann;
- Anordnen des Ballons (40), der näherungsweise die Form eines Zylinders hat in einem nicht expandierten Zustand auf dem Umfang des Katheterschlauches (42) benachbart zu der vorab festgelegten Position des Ballons (40);
**dadurch gekennzeichnet, dass** das Verfahren den weiteren Schritt umfasst,
- beide Enden des Ballons (40) so in axialer Richtung in einem in der axialen Richtung gestreckten Zustand fest zu montieren, dass die vorab festgelegte Position des Katheterschlauches (42) bedeckt wird.

2. Verfahren nach Anspruch 1, wobei
- der Ballon (40) in axialer Richtung zwei Endteile, die einen Innendurchmesser haben, der vor dem Strecken im Wesentlichen gleich einem Außendurchmesser eines montierten Katheters ist, und ein Zwischenteil aufweist, das einen Außendurchmesser hat, der vor dem Strecken im Wesentlichen größer als der Außendurchmesser der beiden in axialer Richtung vorhandenen Endteile ist; und
- die beiden in axialer Richtung vorhandenen Endteile auf dem Katheterschlauch (42) in einer Weise fest montiert werden, dass durch das Strecken des Zwischenteils Beim Strecken in axialer Richtung ein in etwa einheitlicher zylinderförmiger Ballon (40) gebildet werden kann.

3. Verfahren nach Anspruch 1, wobei
- der Ballon (40) in axialer Richtung zwei Endteile, die einen Innendurchmesser haben, der vor dem Strecken in axialer Richtung im Wesentlichen gleich groß wie der Außendurchmesser eines montierten Katheters ist, und ein Zwischenteil aufweist, das einen Innendurchmesser hat, der vor dem Strecken im Wesentlichen größer als der Außendurchmesser der beiden in axialer Richtung vorhandenen Endteile ist, und wobei
- die beiden in axialer Richtung vorhandenen Endteile so auf dem Katheterschlauch (42) montiert werden, dass durch das Strecken des Zwischenteils ein näherungsweise einheitlicher zylinderförmiger Ballon (40) gebildet werden kann, wenn er gestreckt wird.

## Revendications

1. Méthode de montage en position fixe d'un ballonnet (40) sur un tube cathéter (42), un ou plusieurs ballonnets (40) étant disposés près d'un bord supérieur d'insertion du tube cathéter (42) et disposés séparément dans une direction axiale, comprenant les étapes consistant à :
- fournir un cathéter (42) sur lequel ledit ballonnet (40) peut être positionné et monté en position fixe dans une position désignée ;
- placer le ballonnet (40) ayant approximativement la forme d'un cylindre dans un état non dilaté à la périphérie dudit cathéter (42) adjacent à la position prédéterminée dudit ballonnet (40) ;
**caractérisée en ce que** la méthode comprend une étape supplémentaire qui consiste à :
- monter en position fixe les deux extrémités dudit ballonnet (40) dans la direction axiale dans un état allongé dans la direction axiale afin de recouvrir ladite position prédéterminée dudit cathéter (42).

2. Méthode selon la revendication 1, dans laquelle
- ledit ballonnet (40) possède une partie située aux deux extrémités dans la direction axiale qui présente un diamètre intérieur sensiblement égal à un diamètre extérieur d'un cathéter monté avant l'allongement et une partie intermédiaire qui présente un diamètre extérieur sensiblement supérieur au diamètre extérieur de ladite partie située aux deux extrémités dans la direction axiale avant l'allongement de celle-ci ; et
- ladite partie située aux deux extrémités dans la direction axiale est montée en position fixe sur ledit cathéter (42) de sorte qu'un ballonnet de forme approximativement cylindrique uniforme (40) puisse être formé en allongeant ladite partie intermédiaire lors de l'allongement dans la direction axiale.

3. Méthode selon la revendication 1, dans laquelle
- ledit ballonnet (40) possède une partie située aux deux extrémités dans la direction axiale qui présente un diamètre intérieur sensiblement égal au diamètre extérieur d'un cathéter monté avant l'allongement dans la direction axiale et une partie intermédiaire qui présente un diamètre intérieur sensiblement supérieur au diamètre extérieur de ladite partie située aux deux extrémités dans la direction axiale avant l'allongement ; et
- ladite partie située aux deux extrémités dans la direction axiale est montée en position fixe sur ledit cathéter (42) de sorte qu'un ballonnet de forme sensiblement cylindrique uniforme (40) puisse être formé en allongeant ladite partie intermédiaire lors de l'allongement de celle-ci.
